# EUROPÄISCHE PATENTANMELDUNG

(11) **EP 2 923 718 A1**
(43) Veröffentlichungstag der Anmeldung: **30.09.2015**
(21) Anmeldenummer: 14162244.9
(22) Anmeldetag: 28.03.2014
(51) Int. Cl.: A61M 5/34

(54) **Zahnärztliche Spritze und Kanülensockel**

(71) Anmelder: Transcodent GmbH & Co. KG, 24149 Kiel (DE)
(72) Erfinder: Rolle, Phillip, 24536 Neumünster (DE)
(74) Vertreter: Glawe, Delfs, Moll

(57) **Zusammenfassung**

Die Erfindung betrifft eine zahnärztliche Spritze zum Ausbringen flüssiger oder pastöser Stoffe. Die Spritze umfasst einen von einem Spritzengehäuse (30) umschlossenen Innenraum (16), mit einem in dem Innenraum (16) geführten Kolben (31) und einer an den Innenraum (16) angeschlossenen Kanüle (26). Die Kanüle (26) ist in einem Aufnahmekanal (25) eines Kanülensockels (14) gehalten. Der Kanülensockel (14) ist auf eine Ansatzspitze (18) des Spritzengehäuses (30) aufgesteckt ist. Erfindungsgemäß ist der Aufnahmekanal (25) des Kanülensockels (14) in Bezug zur Längsachse (15) der Spritze geneigt. Die Erfindung betrifft außerdem einen Kanülensockel (14) für eine solche Spritze. Da der Aufnahmekanal (25) in die gewünschte Richtung weist, kann die Kanüle (26) kürzer ausfallen als bei bekannten Spritzen. Es ist weniger Kraft erforderlich, um die Substanz aus der Spritze durch die Kanüle (26) hindurch zu bewegen.

## Beschreibung

Die Erfindung betrifft eine zahnärztliche Spritze zum Ausbringen einer flüssigen oder pastösen Substanz. Die Spritze umfasst einen von einem Spritzengehäuse umschlossenen Innenraum. In dem Innenraum ist ein Kolben geführt. An den Innenraum ist eine Kanüle angeschlossen. Die Kanüle ist in einem Aufnahmekanal des Kanülensockels gehalten. Der Kanülensockel ist auf eine Ansatzspitze des Spritzengehäuses aufgesteckt. Die Erfindung betrifft außerdem einen Kanülensockel für eine solche Spritze.

Solche Spritzen können dazu dienen, eine flüssige oder pastöse Substanz an einer bestimmten Stelle dosiert auszubringen. So kann etwa eine Dentalmasse in eine Kavität eines Zahns im Mundraum eines Patienten eingebracht werden, um dort auszuhärten. Die Substanz, die anfänglich in dem von dem Spritzengehäuse umschlossenen Innenraum aufgenommen ist, wird unter Druck gesetzt, indem der Spritzenzylinder weiter in den Innenraum der Spritze hineingedrückt wird. Die Substanz bewegt sich aus dem Innenraum heraus durch die Kanüle hindurch und tritt am vorderen Ende der Kanüle aus.

Oftmals ist es vom Benutzer der Spritze gewünscht, dass das vordere Ende der Kanüle in Bezug zur Längsachse der Spritze geneigt ist. Dies ist beispielsweise dann von Vorteil, wenn der Ausbringungsort schwer zugänglich ist, wie es bei einer Kavität eines Zahns häufig der Fall ist. Um eine Neigung des vorderen Endes der Kanüle relativ zur Längsachse der Spritze zu erreichen, ist es bekannt, die Kanüle um einen gewünschten Winkel zu verbiegen. Nachteilig daran ist, dass beim Biegen der Kanüle ein bestimmter Mindestabstand zum Kanülensockel nicht unterschritten werden kann. Die Kanüle erstreckt sich also ausgehend von dem in dem Aufnahmekanal gehaltenen Abschnitt noch ein Stück geradlinig, bevor die Biegung sich anschließt. Dies bedingt eine gewisse Mindestlänge der Kanüle, was wiederum zur Folge hat, dass beim Ausbringen der Substanz eine erhöhte Reibung zwischen der Substanz und dem Innenwand der Kanüle überwunden werden muss. Außerdem vermindert sich beim Biegen der Kanüle regelmäßig der Innendurchmesser, was ebenfalls den Reibungswiderstand vergrößert. Zum Betätigen der Spritze ist also ein erhöhter Krafteinsatz erforderlich.

Es ist die Aufgabe der vorliegenden Erfindung, eine Spritze vorzustellen, mit der Zahnkavitäten gut erreicht werden können und die mit verminderter Kraft bedient werden kann. Gelöst wird die Aufgabe durch die Merkmale der unabhängigen Ansprüche. Vorteilhafte Ausführungsformen sind in den Unteransprüchen angegeben. Erfindungsgemäß ist der Aufnahmekanal des Kanülensockels in Bezug zur Längsachse der Spritze geneigt.

Zunächst werden einige Begriff erläutert. Zahnärztliche Spritze bedeutet, dass die Spritze zur Verwendung durch einen Zahnarzt geeignet ist. Wenn eine Kanüle an den Innenraum einer Spritze angeschlossen ist, gibt es eine Verbindung zwischen der Kanüle und dem Innenraum, so dass eine Substanz aus dem Innenraum sich zu der Kanüle und durch die Kanüle hindurch bewegen kann.

Durch die erfindungsgemäße Neigung des Aufnahmekanals in Bezug zur Längsachse der Spritze erstreckt sich die Kanüle direkt vom Kanülensockel in eine Richtung, die einen guten Zugang zu beispielsweise einer Zahnkavität ermöglicht. Es ist also nicht notwendig, die Kanüle diesem Zweck zu verbiegen. Es kann also eine gerade Kanüle in den Aufnahmekanal eingesetzt werden, welche dann bereits den gewünschten Winkel mit der Längsachse der Spritze einschließt. Die Erfindung hat erkannt, dass auf diese Weise eine deutlich kürzere Kanüle verwendet werden kann. Insbesondere kann der Abschnitt der Kanüle eingespart werden, welcher bei vorbekannten Kanülen für die Verbiegung notwendig war. Durch die verkürzte Kanüle muss ein Benutzer weniger Kraft zum Ausbringen der Substanz aufwenden, da der zum Ausbringen des Materials notwendige Auspressdruck durch die kleinere Reibung verringert ist.

In einer Ausführungsform schließt der Aufnahmekanal einen Winkel zwischen 30° und 75°, bevorzugt einen Winkel zwischen 40° und 50°, weiter bevorzugt einen Winkel von 45° mit der Längsachse der Spritze ein. Diese Winkelbereiche erlauben dem Benutzer im Falle schwer zugänglicher Ausbringungsorte eine einfache Bedienung der Spritze.

Der Kanülensockel kann eine Innenfläche aufweisen, die an die Außenfläche der Ansatzspitze angepasst ist, so dass die Innenfläche in einem Kontaktabschnitt flächig auf der Außenfläche der Ansatzspitze aufliegt. Bezeichnet man die Strecke vom hinteren Ende des Kanülensockels bis zum Eintrittsende der Kanüle als Länge des Kanülensockels, so kann sich der Kontaktabschnitt über mindestens 40%, vorzugsweise mindestens 60%, weiter vorzugsweise mindestens 80% der Länge des Kanülensockels erstrecken. Durch den Kontaktabschnitt kann der Innenraum des Kanülensockels abgedichtet sein, so dass es für die Substanz, die sich aus dem Innenraum der Spritze in den Kanülensockel bewegt, nur den Weg durch die Kanüle gibt. Die Ansatzspitze des Spritzengehäuses kann als Luer-Konus geformt sein. Der Kanülensockel kann eine zu dem Luer-Konus passende Aufnahme bilden.

Der Kanülensockel kann lösbar mit der Ausbringspitze des Spritzengehäuses verbunden sein. Es kann ein einfacher und abdichtender Reibschluss zwischen der Ansatzspitze des Spritzengehäuses und dem Kanülensockel bestehen.

Möglich ist auch eine zusätzliche Sicherung für die Verbindung zwischen dem Kanülensockel und der Ansatzspitze. Der Kanülensockel kann zu diesem Zweck ein Gewinde aufweisen, das mit einem am vorderen Ende des Spritzengehäuses angeordneten Gegengewinde in Eingriff gebracht werden kann. Das Gewinde des Kanülensockels kann ein Außengewinde sein, das Gewinde des Spritzengehäuses kann ein Innengewinde sein. Mit Hilfe von Gewinde und Gegengewinde kann der Kanülensockel sicher am vorderen Ende der Spritze befestigt werden.

Die Ausbringspitze des Spritzengehäuses hat eine Austrittsöffnung, durch die die Substanz hindurchtritt, wenn der Kolben in das Spritzengehäuse hineingedrückt wird. Um möglichst wenig Ausschussmengen von der Substanz zu haben, wäre es wünschenswert, dass die Substanz unmittelbar von der Austrittsöffnung der Ansatzspitze in die Eintrittsöffnung der Kanüle übergeht. Verteilt sich die Substanz zunächst in einem Innenraum des Kanülensockels, bevor sie in die Kanüle eintreten kann, ergeben sich Ausschussmengen, weil diese Anteile der Substanz nicht sinnvoll genutzt werden können. Allerdings hat sich gezeigt, dass angesichts des Winkels zwischen der Kanüle und der Achse der Spritze bereits kleine Ungenauigkeiten zu Fehlfunktionen führen können, weil kein hinreichender Durchgang von der Ansatzspitze zur Kanüle zur Verfügung steht.

Mit der Erfindung wird deswegen vorgeschlagen, im Inneren des Kanülensockels einen Transferraum vorzusehen, den die Substanz zwischen der Austrittsöffnung der Ansatzspitze und der Eintrittsöffnung der Kanüle durchquert. Damit nimmt man zwar gewisse Ausschussmengen der Substanz in Kauf, das Risiko von Fehlfunktionen wird aber erheblich vermindert.

Der Transferraum kann beispielsweise ein Volumen zwischen 2 mm³ und 40 mm³, vorzugsweise ein Volumen zwischen 10 mm³ und 30 mm³ haben. Das hintere Ende des Transferraums kann von der die Austrittsöffnung umgebenden Stirnfläche der Ansatzspitze gebildet sein. Der äußere Rand der Stirnfläche kann dichtend mit der Innenfläche des Kanülensockels abschließen, so dass dort keine Substanz eintreten kann.

Das innere Ende des Aufnahmekanals, der die Kanüle hält, kann in einer Stirnwand des Transferraums münden. Die Angaben vorne und hinten beziehen sich auf die Längsrichtung der Spritze, also die Richtung, in der der Kolben in das Spritzengehäuse eingeführt wird. Das innere Ende des Aufnahmekanals kann zentral in der Stirnwand münden, also im Bereich der Längsachse der Spritze.

Der Spritzensockel kann aus einem Kunststoffmaterial bestehen. Vorzugsweise ist der Kanülensockel als einstückiges Spritzgussteil gefertigt. Die Kanüle ist auf geeignete Weise in dem Aufnahmekanals des Kanülensockels fixiert. Möglich ist es beispielsweise, die Kanüle direkt beim Herstellen des Kanülensockels zu umspritzen. Auch eine Klebeverbindung zwischen der Kanüle und dem Spritzensockel kommt in Betracht. Die Kanüle kann beispielsweise eine Länge zwischen 8 mm und 14 mm, vorzugsweise zwischen 9 mm und 12 mm haben. Damit ist die Kanüle um etwa 30% kürzer als die bisherigen Kanülen, deren vorderes Ende umgebogen wird. Die erforderliche Kraft, um die Substanz durch die Kanüle hindurchzubewegen, vermindert sich entsprechend. Die Kanüle besteht vorzugsweise aus Metall, das weiter vorzugsweise weichgeglüht ist.

Gegenstand der Erfindung ist außerdem ein Kanülensockel für eine solche Spritze. Der Kanülensockel umfasst eine in Längsrichtung ausgerichtete Aufnahme für eine Ansatzspitze eines Spritzengehäuses. Der Kanülensockel umfasst außerdem einen Aufnahmekanal, in dem eine Kanüle gehalten ist. Der Aufnahmekanal ist relativ zur Längsrichtung des Kanülensockels geneigt. Die Aufnahme für die Ansatzspitze kann einen an einer Innenfläche des Kanülensockels angeordneten Kontaktabschnitt aufweisen, der dazu bestimmt ist, auf einer Ansatzspitze eines Spritzengehäuses aufzuliegen. Der Kontaktabschnitt kann sich über mindestens 40%, vorzugsweise mindestens 60%, weiter vorzugsweise mindestens 80% der Länge des Kanülensockels erstrecken. Der Kanülensockel kann mit weiteren Merkmalen fortgebildet werden, die im Zusammenhang der erfindungsgemäßen Spritze beschrieben sind.

Nachfolgend wird die Erfindung anhand einer vorteilhaften Ausführungsform unter Bezugnahme auf die beigefügten Zeichnungen erläutert. Es zeigen:
- Figur 1:: eine erfindungsgemäße Spritze in einer Seitenansicht; und
- Figur 2:: eine vergrößerte Darstellung von Teilen der Spritze aus Fig. 1.

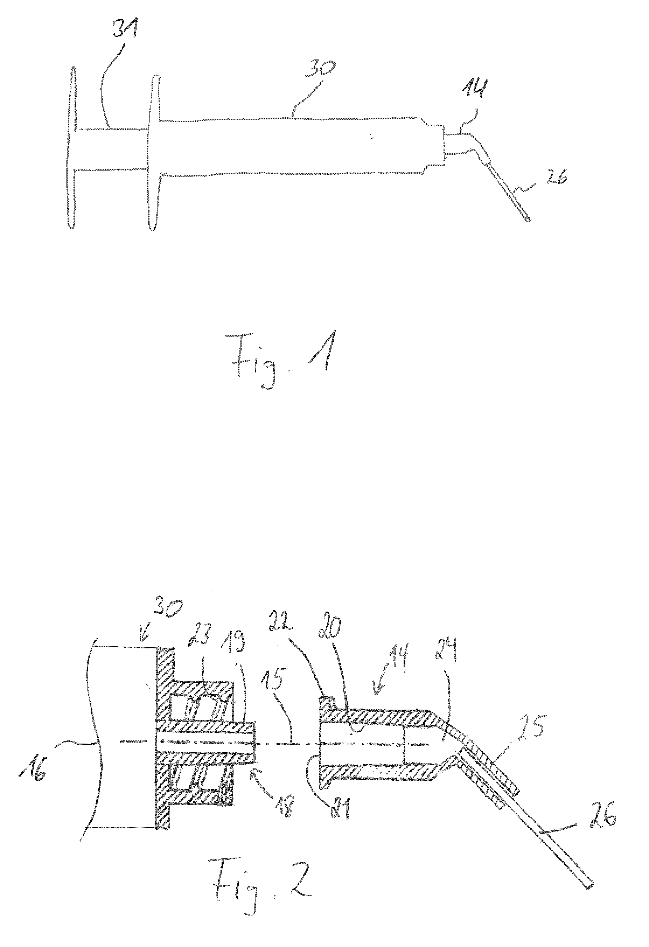

Eine erfindungsgemäße zahnärztliche Spritze in Fig. 1 umfasst ein Spritzengehäuse 30 und einen in den Innenraum des Spritzengehäuses 30 eingeführten Kolben 31. Der Kolben 31 dichtet den Innenraum des Spritzengehäuses 30 ab. Durch weiteres Einführen des Kolbens 31 in das Spritzengehäuse 30 vermindert sich das Volumen im Innenraum des Spritzengehäuses 30 und eine in dem Spritzengehäuse enthaltene Substanz tritt am vorderen Ende der Spritze aus. Die Spritze kann beispielsweise dazu verwendet werden, eine Dentalmasse in einer Zahnkavität im Mund eines Patienten gezielt und dosiert auszubringen.

An das vordere Ende Spritzengehäuses 30 ist ein Kanülensockel 14 angeschlossen, der in einem Aufnahmekanal eine Kanüle 26 hält. Der Kanülensockel 14 bildet eine durchgehende Verbindung zwischen dem Innenraum des Spritzengehäuses 30 und der Kanüle 26, so dass die Substanz aus dem Spritzengehäuse 30 in die Kanüle 26 übertreten und am vorderen Ende der Kanüle 26 austreten kann.

Der Kanülensockel 14 ist in Figur 2 zur besseren Übersicht von der Spritze 13 getrennt, er kann aber entlang der strichpunktiert angedeuteten Längsachse 15 der Spritze 13 auf diese aufgesetzt werden.

Beim Aufsetzen des Kanülensockels 14 auf die Spritze 13 wird eine Ansatzspitze 18 der Spritze 13 durch die Anschlussöffnung 21 in den Kanülensockel 14 eingeführt. Dabei kommt es zu einem Reibschluss zwischen einer Außenfläche 19 der Ansatzspitze 18 und einer Kontaktfläche 20 an der Innenseite des Kanülensockels 14. Die Ansatzspitze 18 ist als Luer-Konus und die Kontaktfläche 20 als zum Luer-Konus passende Gegenfläche ausgestaltet. Die Kontaktfläche 20 erstreckt sich in Längsrichtung über 60% der Länge des Kanülensockels 14, so dass ein guter Reibschluss hergestellt werden kann. Außerdem weist der Kanülensockel 14 an seinem hinteren Ende ein Außengewinde 22 auf, das mit einem korrespondierenden Innengewinde 23 am vorderen Ende der Spritze 13 in Eingriff gebracht werden kann.

An seinem vorderen Ende weist der Innenraum einen Transferraum 24 auf. Der Transferraum 24 geht in den Aufnahmekanal 25 der Kanüle 26 über. Die Kanüle 26 ist in den Aufnahmekanal 25 eingeführt und mit einem Klebstoff fixiert. Die Achse des Aufnahmekanals 25 ist um 45° zur Längsachse 15 der Spritze geneigt. Die Kanüle 26 hat eine Länge von 11 mm und ist damit um ca. 30 % kürzer als bisherige Kanülen, die zum Umbiegen bestimmt sind.

Bei Betätigung der Spritze 13 gelangt die Dentalmasse zunächst in den Transferraum 24 des Kanülensockels 14 und wird dann in Richtung der Eintrittsöffung der Kanüle 26 gedrückt.

## Patentansprüche

1. Zahnärztliche Spritze zum Ausbringen flüssiger oder pastöser Stoffe mit einem von einem Spritzengehäuse (30) umschlossenen Innenraum (16), mit einem in dem Innenraum (16) geführten Kolben (31) und einer an den Innenraum (16) angeschlossenen Kanüle (26), wobei die Kanüle (26) in einem Aufnahmekanal (25) eines Kanülensockels (14) gehalten ist und wobei der Kanülensockel (14) auf eine Ansatzspitze (18) des Spritzengehäuses (30) aufgesteckt ist, **dadurch gekennzeichnet, dass** der Aufnahmekanal (25) des Kanülensockels (14) in Bezug zur Längsachse (15) der Spritze geneigt ist.

2. Zahnärztliche Spritze nach Anspruch 1, **dadurch gekennzeichnet, dass** der Aufnahmekanal (25) einen Winkel zwischen 30° und 75°, vorzugsweise einen Winkel zwischen 40° und 50°, weiter vorzugsweise einen Winkel von 45° mit der Längsachse (15) der Spritze einschließt.

3. Zahnärztliche Spritze nach einem der Ansprüche 1 oder 2, **dadurch gekennzeichnet, dass** der Kanülensockel (14) eine Innenfläche aufweist, die dazu ausgebildet ist als Kontaktabschnitt (20) auf der Ansatzspitze (19) aufzuliegen.

4. Zahnärztliche Spritze nach Anspruch 3, **dadurch gekennzeichnet, dass** der der Kontaktabschnitt (20) sich über mindestens 40%, vorzugsweise mindestens 60%, weiter vorzugsweise mindestens 80% der Länge des Kanülensockels (14) erstreckt.

5. Zahnärztliche Spritze nach Anspruch 3 oder 4, **dadurch gekennzeichnet, dass** in dem Kontaktabschnitt (20) ein dichtender Reibschluss zwischen dem Kanülensockel (14) und der Ansatzspitze (19) besteht.

6. Zahnärztliche Spritze nach einem der Ansprüche 1 bis 5, **dadurch gekennzeichnet, dass** der Kanülensockel (14) durch eine Gewindeverbindung (22, 23) an dem Spritzengehäuse (30) gesichert ist.

7. Zahnärztliche Spritze nach einem der Ansprüche 1 bis 6, **dadurch gekennzeichnet, dass** im Inneren des Kanülensockels (14) ein Transferraum (24) angeordnet ist.

8. Zahnärztliche Spritze nach Anspruch 7, **dadurch gekennzeichnet, dass** der Transferraum (24) ein Volumen zwischen 2 mm³ und 40 mm³, vorzugsweise ein Volumen zwischen 10 mm³ und 30 mm³ hat.

9. Zahnärztliche Spritze nach einem der Ansprüche 1 bis 8, **dadurch gekennzeichnet, dass** das innere Ende des Aufnahmekanals (25) in einer Stirnwand des Transferraums (24) mündet.

10. Zahnärztliche Spritze nach Anspruch 9, **dadurch gekennzeichnet, dass** das innere Ende des Aufnahmekanals (25) zentral in der Stirnwand mündet.

11. Kanülensockel für eine zahnärztliche Spritze nach einem der Ansprüche 1 bis 10, umfassend eine in Längsrichtung (15) ausgerichtete Aufnahme (20) für eine Ansatzspitze (18) eines Spritzengehäuses (30) und einen Aufnahmekanal (25), in dem eine Kanüle (26) gehalten ist, **dadurch gekennzeichnet, dass** der Aufnahmekanal (25) bezogen auf die Längsrichtung (15) geneigt ist.
